(19) Europäisches Patentamt
European Patent Office
Office européen des brevets

(11) Publication number: **0 648 780 A1**

# (12) EUROPEAN PATENT APPLICATION

(21) Application number: **94112916.5**

(51) Int. Cl.[6]: **C07K 5/06**, A61K 38/55

(22) Date of filing: **18.08.94**

(30) Priority: **26.08.93 US 112155**

(43) Date of publication of application: **19.04.95 Bulletin 95/16**

(84) Designated Contracting States: **AT BE CH DE DK ES FR GB GR IE IT LI LU MC NL PT SE**

(71) Applicant: **BRISTOL-MYERS SOUIBB COMPANY**
**P.O. Box 4000**
**Princeton, NJ 08543-4000 (US)**

(72) Inventor: **Kimball, Spencer D.**
**13 Charred Oak lane**
**E. Windsor, NJ 08520 (US)**
Inventor: **Hall, Steven E-**
**102 Nuttal Place**
**Chapel Hill, NC (US)**
Inventor: **Han, Wen-Ching**
**2062 East Wellington Rd.**
**Newtown, PA 18966 (US)**

(74) Representative: **Josif, Albert, Dr.-Ing. et al**
**Baaderstrasse 3**
**D-80469 München (DE)**

(54) **Heterocyclic thrombin inhibitors.**

(57) Heterocyclic thrombin inhibitors are provided which have the structure

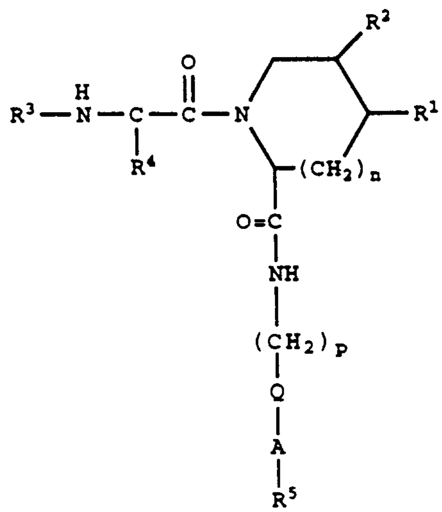

including all stereoisomers thereof
wherein n is 0, l or 2;
p is 0, l or 2;
Q is a single bond or C = O;
A is aryl or cycloalkyl, or an azacycloalkyl ring or an azaheteroalkyl ring;
$R^5$ is guanidine, amidine or aminomethyl, but when A is azacycloalkyl or azaheteroalkyl, $R^5$ is amidine;
$R^1$ and $R^2$ are independently hydrogen, lower alkyl, cycloalkyl, aryl, hydroxy alkoxy, keto, thioketal, thioalkyl, thioaryl, amino or alkylamino; or $R^1$ and $R^2$ together with the carbons to which they are attached form a

EP 0 648 780 A1

cycloalkyl aryl, or heteroaryl ring;

$R^4$ is hydrogen, hydroxyalkyl, aminoalkyl, amidoalkyl, alkyl, cycloalkyl, aryl, arylalkyl, alkenyl, alkynyl, arylalkoxyalkyl, or an amino acid side chain, either protected or unprotected; and

$R^3$ is hydrogen,

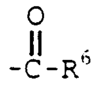 ,

or $-CO_2R^6$ (wherein $R^6$ is lower alkyl, aryl, arylalkyl or cycloheteroalkyl);

including pharmaceutically acceptable salts thereof.

The present invention relates to heterocyclic compounds which are thrombin inhibitors and thus useful in inhibiting formation of thrombi.

The heterocyclic thrombin inhibitors of the invention have the structure I

I.

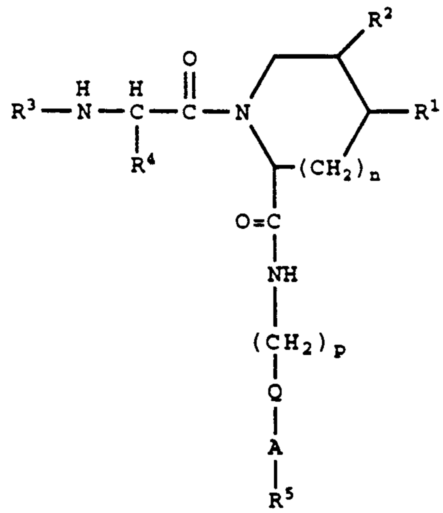

including all stereoisomers thereof, and including all pharmaceutically acceptable salts thereof; wherein n is 0, l or 2;

p is 0, l or 2;

Q is a single bond or

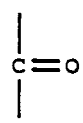

;

A is aryl or cycloalkyl, or an azacycloalkyl ring A of 3 to 7 carbons in the ring or an azaheteroalkyl ring A of 4 to 6 carbons in the ring, as given by the structure

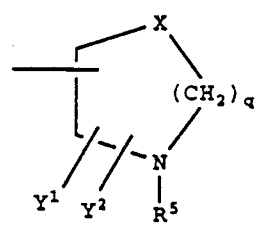

X is $CH_2$, O, S or NH;

q = 0, l, 2, 3 or 4 if X = $CH_2$;

q = 2, 3 or 4 if X = O, S, NH; and

$Y^1$ and $Y^2$ are independently H, alkyl, halo or keto;

$R^5$ is guanidine, amidine or aminomethyl;

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ together with the carbons to which they are attached form a cycloalkyl, aryl, or heteroaryl ring;

$R^4$ is hydrogen, hydroxyalkyl aminoalkyl, amidoalkyl, alkyl, cycloalkyl, aryl, arylalkyl, alkenyl, alkynyl, arylalkoxyalkyl, or an amino acid side chain, either protected or unprotected; and

$R^3$ is hydrogen,

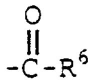 ,

or $-CO_2R^6$ (wherein $R^6$ is lower alkyl, aryl, arylalkyl or cycloheteroalkyl);

with the provisos that where A is aryl or cycloalkyl, $R^5$ is guanidine, amidine or aminomethyl;

where A is azacycloalkyl or azaheteroalkyl, $R^5$ is amidine;

where X is a hetero atom (that is A is azaheteroalkyl), then there must be at least a 2-carbon chain between X and any N atom in the ring A or outside the ring A.

Examples of the A ring (azacycloalkyl or azaheteroalkyl) which may be employed herein include

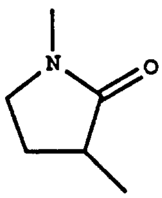 , 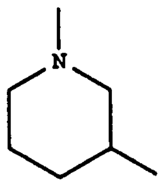 , 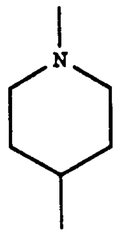 , 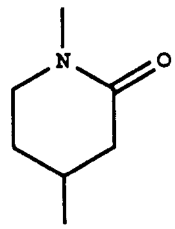 ,

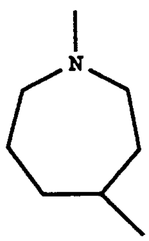 , 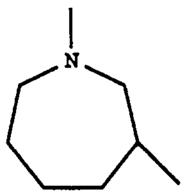 , 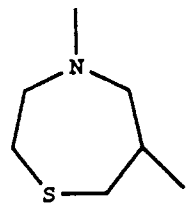 ,

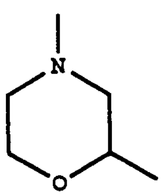 , 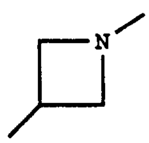 , 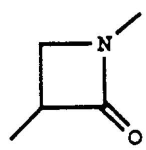 ,

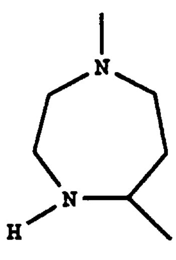 , 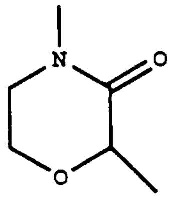 , 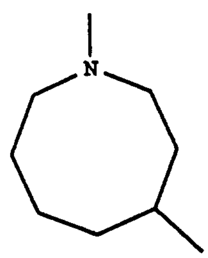

and the like.

The term "lower alkyl" or "alkyl" as employed herein by itself or as part of another group includes both straight and branched chain radicals of up to l8 carbons, preferably l to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including l, 2 or 3 halo substituents, an aryl substituent, an alkylaryl substituent, a haloaryl substituent, a cycloalkyl substituent, an alkylcycloalkyl substituent, an alkenyl substituent, an alkynyl substituent, hydroxy or a carboxy substituent.

The term "cycloalkyl" by itself or as part of another group includes saturated cyclic hydrocarbon groups containing 3 to l2 carbons, preferably 3 to 8 carbons, which include cyclopropyl, cyclobutyl,

cyclopentyl, cyclohexyl, cycloheptyl, cyclooctyl, cyclodecyl and cyclododecyl, any of which groups may be substituted with substituents such as halogen, lower alkyl, alkoxy and/or hydroxy groups.

The term "aryl" or "Ar" as employed herein by itself or as part of another group refers to monocyclic or bicyclic aromatic groups containing from 6 to l0 carbons in the ring portion, such as phenyl, or naphthyl. Aryl (or Ar), phenyl or naphthyl may include substituted aryl, substituted phenyl or substituted naphthyl, which may include I or 2 substituents on either the Ar, phenyl or naphthyl such as lower alkyl, cyano, amino, alkylamino, dialkylamino, nitro, carboxy, carboalkoxy, trifluoromethyl, halogen (Cl Br, I or F), lower alkoxy, arylalkoxy, hydroxy, alkylthio, alkylsulfinyl, alkylsulfonyl, arylthio, arylsulfinyl and/or arylsulfonyl.

The term "aralkyl", "aryl-alkyl" or "aryl-lower alkyl" as used herein by itself or as part of another group refers to lower alkyl groups as discussed above having an aryl substituent, such as benzyl.

The term "lower alkoxy", "alkoxy" or aralkoxy" includes any of the above lower alkyl, alkyl or aralkyl groups linked to an oxygen atom.

The term "halogen" or "halo" as used herein by itself or as part of another group refers to chlorine, bromine, fluorine or iodine with chlorine being preferred.

The term "lower alkenyl" or "alkenyl" as employed herein by itself or as part of another group includes a carbon chain of up to l6 carbons, preferably 3 to l0 carbons, containing one double bond which will be separated from "N" by at least one saturated carbon moiety such as $-(CH_2)_q-$ where q can be I to l4, such as 2-propenyl, 2-butenyl, 3-butenyl, 2-pentenyl, 4-pentenyl and the like, and may include a halogen substituent such as I, Cl, or F.

The term "lower alkynyl" or "alkynyl" as employed herein by itself or as part of another group includes a carbon chain of up to l6 carbons, preferably 3 to l0 carbons, containing one triple bond which will be separated from "N" by at least one saturated carbon moiety such as $-(CH2)_{q'}-$ where q' can be I to l4, such as 2-propynyl, 2-butynyl, 3-butynyl and the like.

The term "heteroaryl" or heteroaromatic by itself or as part of another group refers to a 5- or 6-membered aromatic ring which includes I or 2 hetero atoms such as nitrogen, oxygen or sulfur, such as

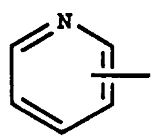 , 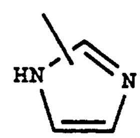 , 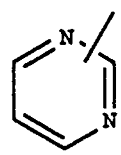 , 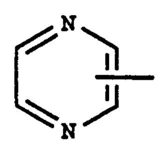 ,

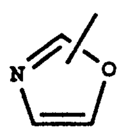 , 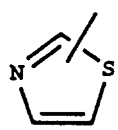 , 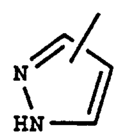 , 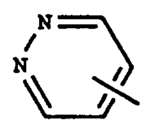 ,

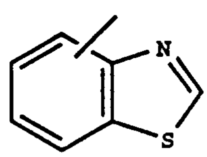 , 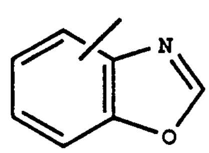 , 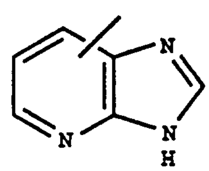 ,

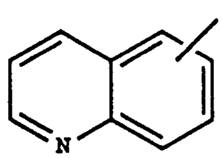 , 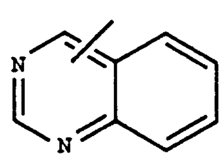 , 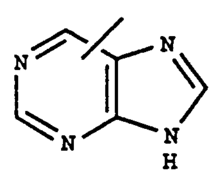

and the like. The heteroaryl rings may optionally be fused to aryl rings defined previously. The hetero-aryl rings may optionally include I or 2 substituents such as halogen (Cl, Br, F or $CF_3$), lower alkyl, lower alkoxy, carboxy, amino, lower alkylamino and/or dilower alkylamino.

The term "cycloheteroalkyl" as used herein refers to a 5-, 6- or 7-membered saturated ring which includes I or 2 hetero atoms such as nitrogen, oxygen and/or sulfur, such as

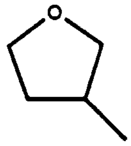 , 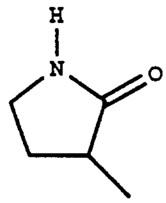 , 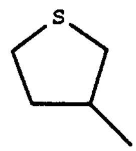 ,

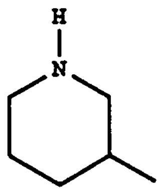 , 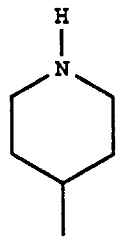 , 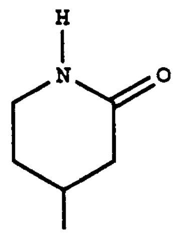 ,

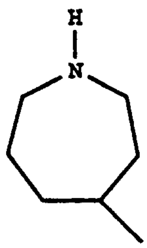 , 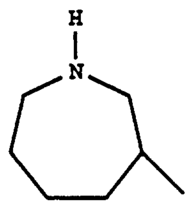

and the like.

The term "amino acid side chain" refers to any of the known alpha-amino acids such as arginine, histidine, alanine, glycine, lysine, glutamine, leucine, valine, serine, homoserine, allothreonine, naphthylalanine isoleucine, phenylalanine and the like.

Preferred are compounds of formula I wherein

n is 0 or l,

$R^3$ is H; $R^4$ is aralkyl or hydroxyalkyl,

$R^1$ and $R^2$ are each H, p is 0 or l,

Q is a single bond, A is an azacycloalkyl ring

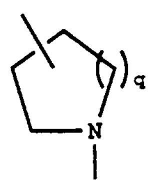

where q is l or 2; $R^5$ is amidine.

Most preferred are compounds of formula I wherein $R^3$ is H, n is 0, $R^1$ and $R^2$ are each H,

$R^4$ is aralkyl such as benzyl,

p is l, Q is a single bond, $AR^5$ is

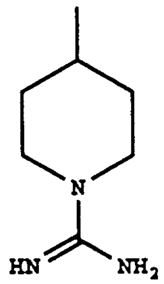

or

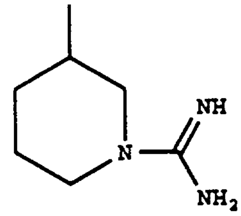

The compounds of formula I of the invention may be prepared according to the following reaction sequences.

The compounds of formula I of the invention wherein A is azacycloalkyl or azaheteroalkyl and $R^5$ is amidine may be prepared according to the following Reaction Sequence I.

The compounds of formula II or III are known in the art or may be prepared by those skilled in the art employing conventional preparatory techniques.

Reaction Sequence I

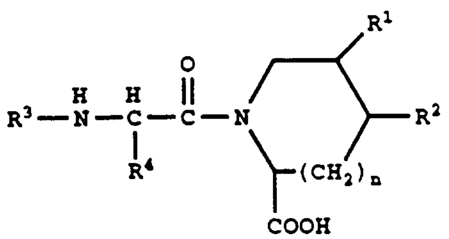

II

+

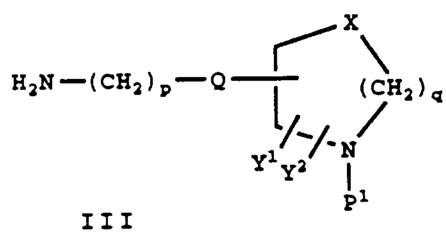

III

(where $P^1$ is a protecting group such as BOC or CBz)

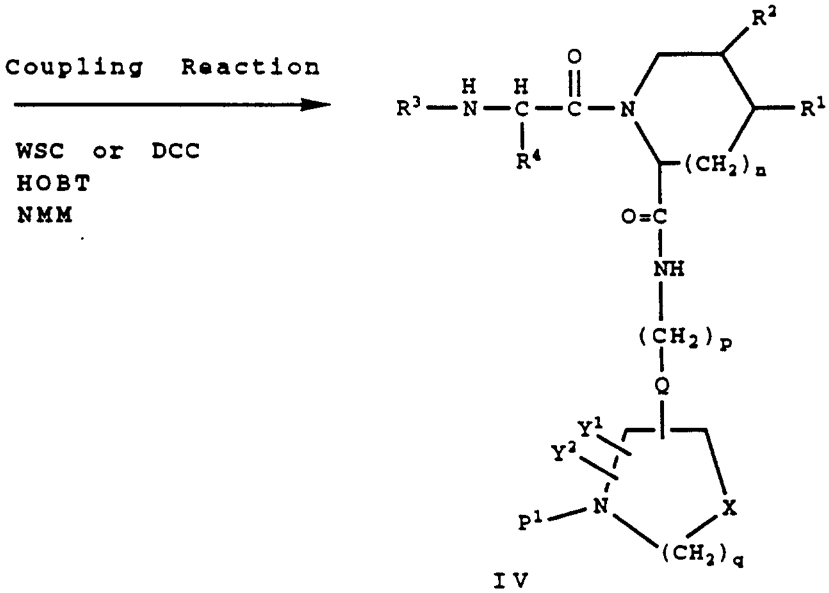
Coupling Reaction
WSC or DCC
HOBT
NMM

IV

IV

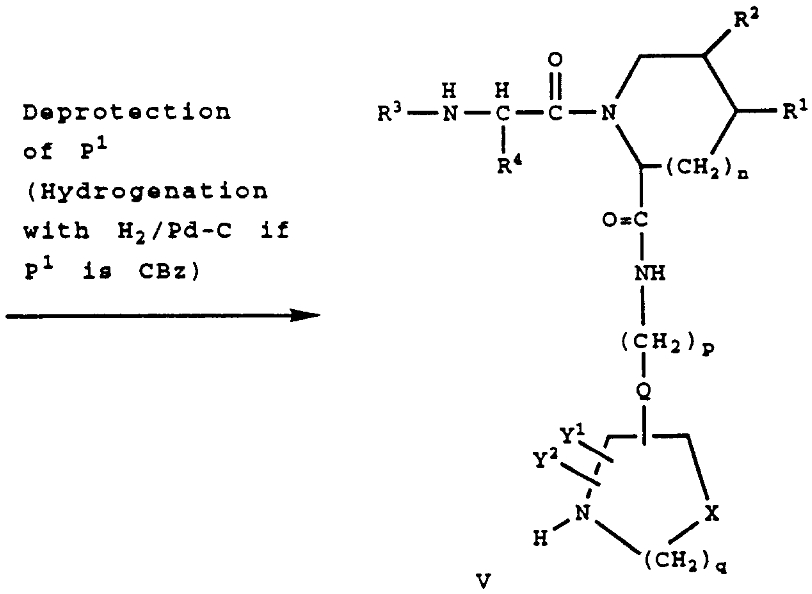
Deprotection
of P1
(Hydrogenation
with H2/Pd-C if
P1 is CBz)

V

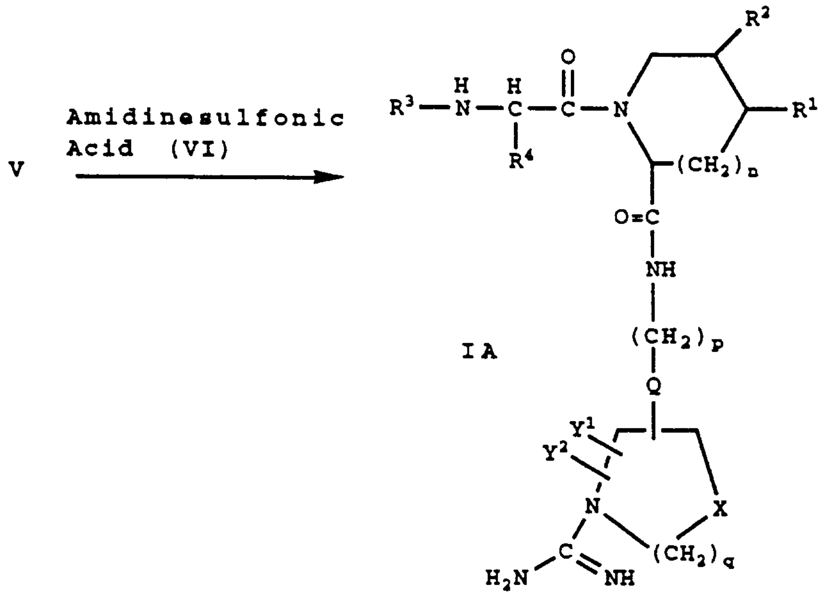


V

IA

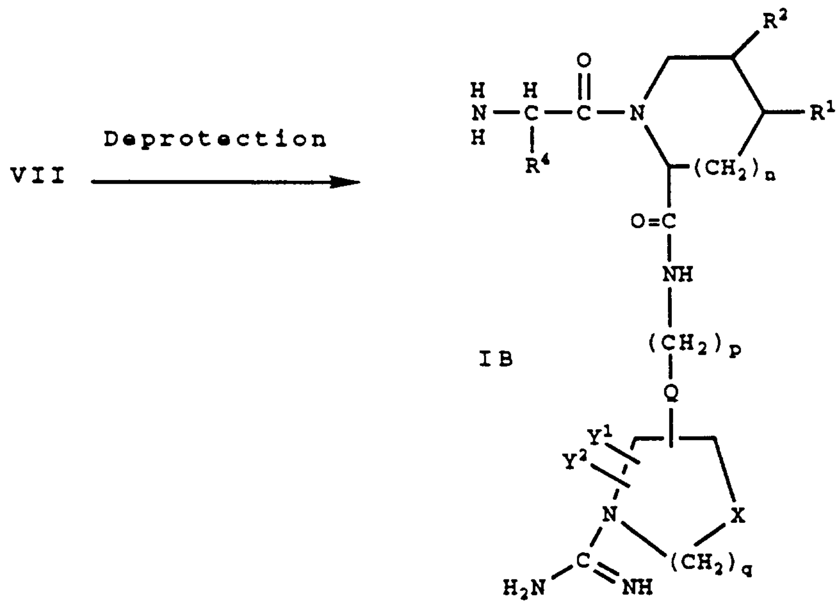


VII

IB

As seen in the above Reaction Sequence I, compounds of formula I wherein A is azacycloalkyl or azaheteroalkyl, are prepared as follows. The protected acid II is made to undergo a carbodiimide coupling reaction with amine III in the presence of ethyl 3-(3-dimethylamino)propyl carbodiimide hydrochloride (WSC) or dicyclohexylcarbodiimide (DCC), and l-hydroxybenzotriazole monohydrate (HOBT), and N-methylmorpholine (NMM), and in the presence of an inert organic solvent such as dimethylformamide (DMF), THF or N-methylpyrrolidone, to form the amide IV. Amide IV is deprotected by treatment with, for example, $H_2$/Pd-C if $P^1$ is CBz, to form amine V. Amine V is treated with amidinesulfonic acid VI in the presence of alcohol solvent, such as ethanol to give cyclic guanidine IA. Guanidine IA is deprotected by treatment with trifluoroacetic acid (if $R^3$ = BOC) with or without the presence of dry inert organic solvent such as dichloromethane, chloroform or THF at temperatures within the range of from about -l5° to about 20°C to form amidine compound of the invention IB.

The compounds of the invention where A is aryl or cycloalkyl and $R^5$ is amidine or guanidine may be prepared according to the following Reaction Sequence II:

Reaction Sequence II

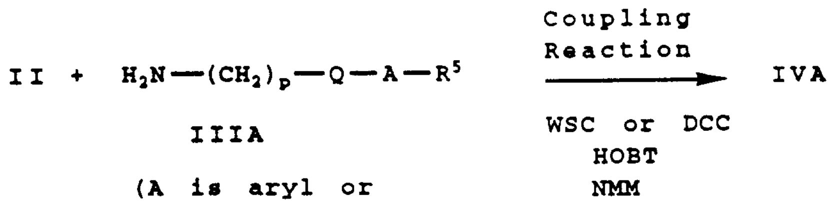


IIIA

(A is aryl or cycloalkyl
$R^5$ is amidine or guanidine)

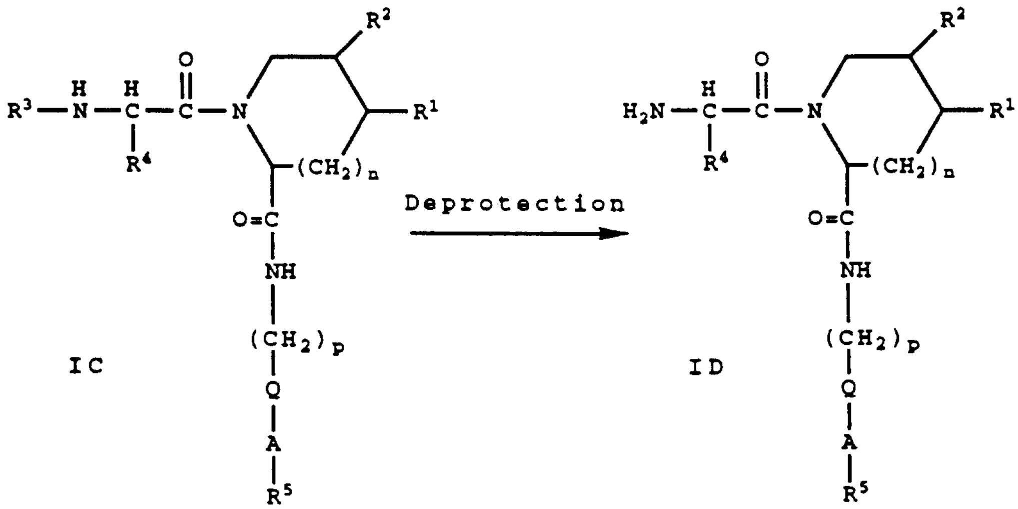


IC

ID

As seen in Reaction Sequence II, compounds of formula I where A is aryl or cycloalkyl (that is $A^1$) and $R^8$ is amidine or guanidine ($R^{8'}$) are prepared as follows. The protected acid II is subjected to a carbodiimide coupling reaction wherein II is treated with protected amine IIIA in the presence of WSC or DCC, and HOBT, and NMM, in the presence of an inert organic solvent such as dimethylformamide, THF or N-methylpyrrolidone, to form amide IC. The amide IC is then dissolved in an alcohol solvent such as ethanol or methanol, to which HCl has been added and the mixture is hydrogenated over Pd-C or $Pd(OH)_2$-C in the case where $R^3$ is carbobenzyloxy, to form compound ID of the invention.

The compounds of formula I of the invention wherein A is aryl or cycloalkyl and $R^5$ is aminomethyl (that is $R^5$) may be prepared according to the following Reaction Sequence III:

Reaction Sequence III

II + $H_2N-(CH_2)_p-Q-A-CH_2NHP^1$

IIIB

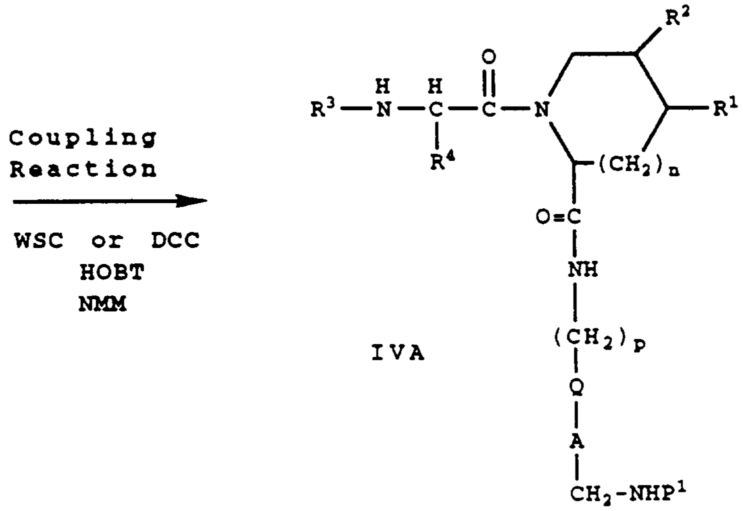


IVA

IVA

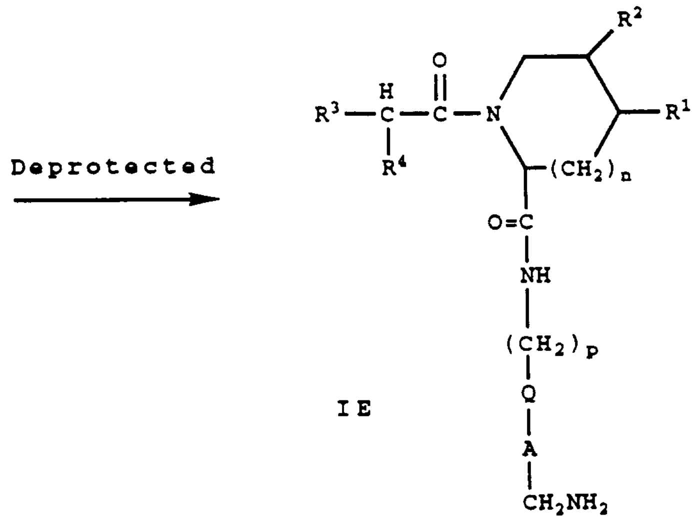


IE

As seen in the above Reaction Sequence III compounds of formula I wherein A is aryl or cycloalkyl and $R^5$ is aminomethyl are prepared as follows. The protected acid II is made to undergo a carbodiimide coupling reaction with protected amino acid IIIB in the presence of ethyl 3-(3-dimethylamino)propyl carbodiimide hydrochloride (WSC) or dicyclohexylcarbodiimide (DCC) and l-hydroxybenzotriazole monohydrate (HOBT), and N-methylmorpholine (NMM), and in the presence of an inert organic solvent such as dimethylformamide (DMF), THF or N-methylpyrrolidone, to form the amide IVA. Amide IVA is deprotected by treatment with trifluoroacetic acid (TFA) when $P^1$ is t-butoxycarbonyl (BOC) or $H_2$-Pd/C when $P^1$ is carbobenzyloxy (CBz), with or without the presence of dry inert organic solvent such as dichloromethane, chloroform or THF, at temperatures within the range of from about -l5° to about 20°C to form amide IE of the invention.

The starting acid II may be prepared according to the following reaction sequence:

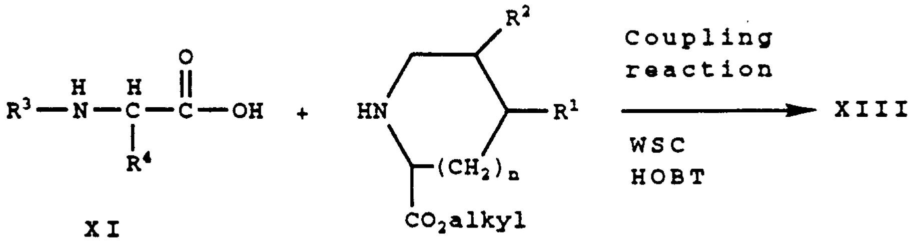


XI

XII

(P=t-butoxycarbonyl (BOC), carbobenzyloxy (CBz))

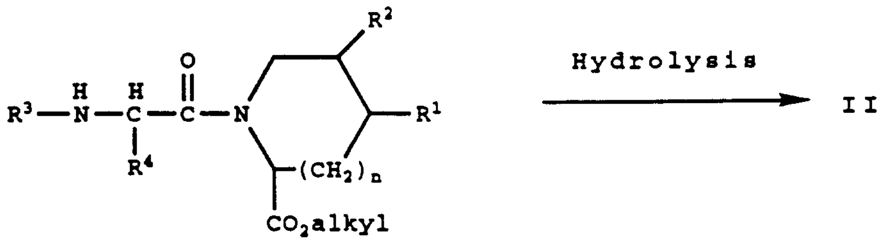


XIII

As seen in the above reaction sequence, compounds of formula II are prepared as follows. The ester XII is made to undergo a carbodiimide couplng reaction with protected amino acid XI in the presence of ethyl 3-(3-dimethylamino)propyl carbodiimide hydrochloride (WSC) or dicyclohexylcarbodiimide (DCC) and l-hydroxybenzotriazole monohydrate (HOBT), and N-methylmorpholine (NMM), and in the presence of an inert organic solvent such as dimethylformamide (DMF), THF or N-methylpyrrolidone, to form the amide XIII. Amide XIII is hydrolyzed by treatment with base such as NaOH, KOH or LiOH to form an alkali metal salt which is neutralized with strong acid such as HCl or oxalic acid to form II.

The compounds of formula I of the invention can be obtained as pharmaceutically acceptable acid addition salts by reacting a free base with an acid, such as hydrochloric, hydrobromic, hydroiodic, nitric, sulfuric, phosphoric, acetic, citric, maleic, succinic, lactic, tartaric, gluconic, benzoic, methanesulfonic, ethanesulfonic, benzenesulfonic, p-toluenesulfonic acid or the like.

The compounds of the present invention are serine protease inhibitors, and in particular may inhibit thrombin, Factor Xa, and/or trypsin. The compounds of the present invention are useful for the treatment or prophylaxis of those processes which involve the production and/or action of thrombin. This includes a number of thrombotic and prothrombotic states in which the coagulation cascade is activated which include, but are not limited to, deep vein thrombosis (DVT), disseminated intravascular coagulopathy (DIC) Kasabach-Merritt syndrome, pulmonary embolism, myocardial infarction, stroke, thromboembolic complications of surgery (such as hip replacement and endarterectomy) and peripheral arterial occlusion. In addition to its effects on the coagulation process, thrombin has been shown to activate a large number of cells (such as neutrophils, fibroblasts, endothelial cells, smooth muscle cells) . Therefore, the compounds of the present invention may also be useful for the treatment or prophylaxis of adult respiratory distress syndrome, septic shock, septicemia, inflammatory responses which include, but are not limited to, edema, acute or chronic atherosclerosis, and reperfusion damage.

The compounds of the invention may also be useful in treating neoplasia/metastasis (in particular those which utilize fibrin) and neurodegenerative diseases such as Alzheimer's disease and Parkinson's disease. In addition, the compounds of the present invention may be useful to prevent restenosis following arterial injury induced by endogenous (rupture of an atherosclerotic plaque) or exogenous (invasive cardiological procedure) events.

The compounds of the present invention may also be used as an anticoagulant in extracorpeal blood circuits, such as those necessary in dialysis and surgery (such as coronary artery bypass surgery).

The compounds of the present invention may also be used in combination with thrombolytic agents, such as tissue plasminogen activator (natural or recombinant), streptokinse, urokinase, prourokinase, anisoylated streptokinase plasminogen activator complex (ASPAC), animal salivary gland plasminogen activators, and the like. The compounds of the present invention may act in a synergistic fashion to prevent

reocclusion following a successful thrombolytic therapy and/or reduce the time to reperfusion. The compounds of the present invention may also allow for reduced doses of the thrombolytic agent to be used and therefore minimize potential hemorrhagic side-effects.

The compounds of the present invention may also be used in combination with other antithrombotic or anticoagulant drugs such as thromboxane receptor antagonists, prostacyclin mimetics, phosphodiesterase inhibitors, fibrinogen antagonists, and the like.

Compounds of the present invention that inhibit trypsin may also be useful for the treatment of pancreatitis.

The compounds of the invention can be administered orally or parenterally to various mammalian species known to be subject to such maladies, e.g., humans, cats, dogs and the like in an effective amount within the dosage range of about 0.l to about l00 mg/kg, preferably about 0.2 to about 50 mg/kg and more preferably about 0.5 to about 25 mg/kg (or from about l to about 2500 mg, preferably from about 5 to about 2000 mg) on a regimen in single or 2 to 4 divided daily doses.

The active substance can be utilized in a composition such as tablet, capsule, solution or suspension containing about 5 to about 500 mg per unit of dosage of a compound or mixture of compounds of formula I. They may be compounded in conventional matter with a physiologically acceptable vehicle or carrier, excipient, binder, preservative, stabilizer, flavor, etc., as called for by accepted pharmaceutical practice.

The following Examples represent preferred embodiments of the present invention. Unless otherwise indicated, all temperatures are expressed in degrees Centigrade.

## Example l

N-[[l-(Aminoiminomethyl)-4-piperidinyl]methyl]-l-D-phenylalanyl-L-prolinamide

A. 4-(Aminomethyl)-N,N'-bis[(l,l-dimethylethoxy)carbonyl]-l-piperidinecarboximidamide

To a stirred solution of 4-aminomethylpiperidine (0.72 g, 6.31 mmol) in 40 mL of toluene was added benzaldehyde (0.78 mL, 6.94 mmol). The reaction solution was refluxed for 18 h and water was removed by a Dean Stark trap. The reaction solution was cooled to room temperature at which time bis-Boc amidinopyrazole (1.96 g, 6.31 mmol) was added. The reaction solution was stirred at room temperature for 48 h and concentrated *in vacuo*. The oily residue was diluted with 15 mL of 1M (aq) KHSO4 solution and stirred at room temperature for 5 h. This aqueous solution was washed with ether (2x20 mL) and basified to pH 12 by the addition of 1N NaOH solution. This basic solution was then saturated with NaCl and extracted with dichloromethane (3x60 mL). The combined dichloromethane extracts were dried ($MgSO_4$), filtered and concentrated *in vacuo* to give 2.10 g (93%) of title amine which was used for the next transformation without further purification.

B. (S*)-N-[[l-[[[(l,l-Dimethylethoxy)carbonyl]amino][[(l,l-dimethylethoxy)carbonyl]imino]methyl]-4-piperidinyl]-methyl]-l-[l-[[(l,l-dimethylethoxy)carbonyl]amino]-2-phenylethyl]-S-prolinamide

To a stirred solution of N-Boc-D-Phe-L-Pro-OH (0.73 g, 2.02 mmol), Part A amine (0.72 g, 2.02 mmol) and 1-hydroxybenzotriazole monohydrate (0.34 g, 2.02 mmol) in 30 mL of DMF was added in order 4-methyl-morpholine (0.66 mL, 6.05 mmol) and ethyl-3-(3-dimethylamino)propyl carbodiimide hydrochloride (0.39 g, 2.02 mmol). The reaction solution was stirred at room temperature for 19 h and concentrated under pump vacuum at 45 °C. The residue was diluted with 100 mL of saturated $NaHCO_3$ solution and extracted with dichloromethane (4x100 mL). The combined dichloromethane extracts were dried ($MgSO_4$), filtered and concentrated *in vacuo*. This was chromatographed on silica gel to give 0.70 g (50%) of title bis-Boc guanidine.

C. N-[[l-(Aminoiminomethyl)-4-piperidinyl]methyl]-l-D-phenylalanyl-L-prolinamide

To a stirred solution of Part B bis-Boc guanidine (0.68 g, 0.97 mmol) in 6.0 mL of dichloromethane was added trifluoroacetic acid (TFA) (6.00 mL, 77.9 mmol). The reaction solution was stirred at room temperature for 3 h and concentrated *in vacuo.* This was purified by prep HPLC to give 310 mg (45%) of title compound.

Example 2

N-[[l-(Aminoiminomethyl)-3-piperidinyl]methyl]-l-D-phenylalanyl-L-prolinamide

A. N-Boc-3-hydroxymethylpiperidine

To a stirred solution of 3-hydroxymethylpiperidine (15.1 g, 131 mmol) and $Et_3N$ (21.9 mL, 158 mmol) in 100 mL of dichloromethane was added dropwise a solution of di-t-butyl dicarbonate (31.5 g, 144 mmol) in 100 mL of dichloromethane over 1 h. The reaction was stirred at room temperature for 18 h and then diluted with 200 mL of dichloromethane. The resulting solution was washed with 1N HCl solution (3x100 mL), saturated $NaHCO_3$ solution (2x100 mL) and brine (1x100 mL). The organic layer was dried ($MgSO_4$), filtered and concentrated *in vacuo* to give N-Boc-3-hydroxymethylpiperidine (27.0 g, 96%).

B. 3-(Azidomethyl)-l-piperidinecarboxylic acid, l,l-dimethylethyl ester

To a stirred solution of Part A N-Boc-3-hydroxymethylpiperidine (27.0 g, 126 mmol) in 150 mL of dry dichloromethane under argon at 0°C was added in order triethylamine (22.7 mL, 163 mmol) and methanesulfonyl chloride (11.7 mL, 151 mmol). The reaction was stirred at room temperature for 1.5 h and diluted with 450 mL of dichloromethane. The reaction was washed with 0°C 1N HCl solution (2x100 mL) and brine (1x100 mL). The dichloromethane layer was dried ($Na_2SO_4$), filtered and concentrated *in vacuo*. The residue was dissolved in 200 mL of DMF and combined with sodium azide (24.5 g, 377 mmol). The mixture was stirred at room temperature for 33 h and the solid was filtered off. The filtrate was concentrated under pump vacuum at 45°C. The residue was partitioned between 400 mL of EtOAc and 10% sodium thiosulfate solution (2x100 mL) and brine (1x100 mL). The EtOAc layer was dried ($MgSO_4$), filtered and concentrated *in vacuo*. Purification was effected by a flash column chromatography on silica gel to give 19.5 g (65%) of title azide.

C. 3-(Aminomethyl)-l-piperidinecarboxylic acid, l,l-dimethylethyl ester

To a stirred solution of Part B azide (19.0g, 79.2 mmol) in 250 mL of methanol under argon was added 10%Pd/C (3.80 g, 20% based on the weight of Part B azide). The atmosphere was replaced with hydrogen by several vacuum-fill cycles. The mixture was stirred at room temperature for 15 h. The catalyst was filtered through a 4μM polycarbonate film and rinsed with methanol (4x30 mL). The filtrate was concentrated *in vacuo* to give 16.3 g (96%) of title amine.

D. N-[[l-[(l,l-Dimethylethoxy)carbonyl]-3-piperidinyl]methyl]-l-[N-[(phenylmethoxy)carbonyl]-D-phenylalanyl]-L-prolinamide

To a stirred solution of Part C amine (2.00 g, 9,35 mmol), N-Cbz-D-Phe-L-Pro (3.70 g, 9.35 mmol), 1-hydroxybenzotriazole monohydrate (1.58 g, 9.35 mmol) and 4-methylmorpholine (3.07 mL, 28.0 mmol) was added ethyl-3-(3-dimethylamino)propyl carbodiimide hydrochloride (1.79 g, 9.35 mmol). The reaction solution was stirred at room temperature for 17 h and concentrated under pump vacuum at 45°C. The residue was dissolved in 360 mL of EtOAc and washed with 1N HCl solution (2x120 mL), saturated $NaHCO_3$ solution (1x120 mL) and brine (1x120 mL). The EtOAc layer was dried ($MgSO_4$), filtered, concentrated *in vacuo* and chromatographed on silica gel to give 1.30g (23%) of title carbamate.

E. N-[[l-(Aminoiminomethyl)-3-piperidinyl]methyl]-l-[N-[(phenylmethoxy)carbonyl]-D-phenylalanyl]-L-prolinamide

To a stirred solution of Part D carbamate (2.30 g, 3.89 mmol) in 10 mL of dry dichloromethane was added 0°C 4N HCl in dioxane (15.0 mL, 60.0 mmol). The solution was stirred at room temperature for 3 h and diluted with 300 mL of ether. The precipitate was filtered off and rinsed with ether (3x30 mL). The precipitate was dried under pump vacuum at room temperature and purified by prep HPLC to give 1.39 g (59%) of intermediate amine•TFA. To a stirred solution of the intermediate amine•TFA salt (500 mg, 0.83 mmol) and diisopropylethyl amine (0.35 mL, 1.98 mmol) in 2.0 mL of DMF was added 1H-pyrazole-1-carboxamidine (133 mg, 0.91 mmol). The reaction solution was stirred at room temperature for 6 h and diluted with 100 mL of ether. The desired oily precipitate was separated from the ether solution and purified by prep HPLC to give 250 mg (47%) of title Cbz-carbamate.

F. N-[[l-(Aminoiminomethyl)-3-piperidinyl]methyl]-l-D-phenylalanyl-L-prolinamide

To a stirred solution of Part E Cbz-carbamate (240 mg, 0.37 mmol) in 10 mL of methanol under argon was added 20%$Pd(OH)_2$/C (48 mg, 20% based on the weight of Part E Cbz carbamate). The atmosphere was replaced with hydrogen by several vacuum-fill cycles. The reaction mixture was stirred at room temperature for 24 h. The catalyst was filtered off and rinsed with methanol (4x20 mL). The filtrate was concentrated *in vacuo*. The residue was dissolved in 50 mL of a solution of 0.1%TFA in water and lyophilized to give 220 mg (82%) of title compound.

Following the procedures of Examples l and 2, the following examples of compounds of the invention may be prepared.

**TABLE**

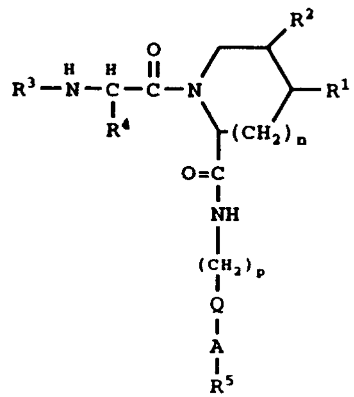

| Example No. | $R^3$ | $R^4$ | $R^2$ | $R^1$ | n | p | Q | A | $R^5$ |
|---|---|---|---|---|---|---|---|---|---|
| 3 | CBZ | $CH_2OH(S)$ | H | $CH_3$ | 1 | 0 | - | 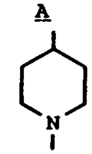 | 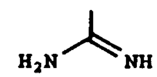 |
| 4 | H | H | OH | H | 0 | 1 | CO | 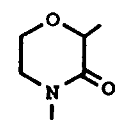 | 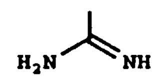 |
| 5 | $C_6H_5CO$ | $-CH_2C_6H_5(R)$ | $OCH_3$ | $CH_3$ | 0 | 2 | - | 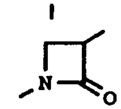 | 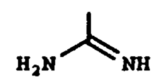 |

| Example No. | $R^3$ | $R^4$ | $R^2$ | $R^1$ | n | p | Q | A | $R^5$ |
|---|---|---|---|---|---|---|---|---|---|
| 6 | $CO_2CH_3$ | $-CH_2C_6H_5$(S) | $CH_3$ | $CH_3$ | 1 | 1 | - | 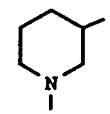 | 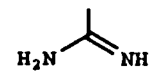 |
| 7 | $CO_2C_6H_5$ | $-CH_2CH_2CONH_2$(S) | H | H | 1 | 0 | - | 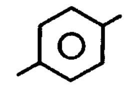 | $-CH_2NH_2$ |
| 8 | $CH_3CO$ | $-CH_2CH_2CONH_2$(R) | H | $CH_3$ | 1 | 1 | CO | 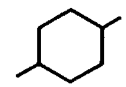 | $-CH_2NH_2$ |
| 9 | 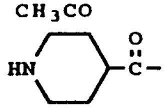 | $-CH(OH)CH_3$(S-Thr) | $-CHCH_2CH_2CH-$ | | 0 | 2 | - | 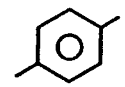 | 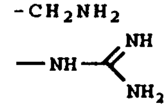 |
| 10 | H | $CH(OH)CH_3$(S-alloThr) | $CH_3$ | H | 1 | 2 | CO | 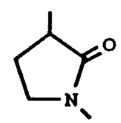 | 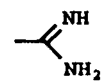 |
| 11 | $C_6H_5CH_2CO$ | 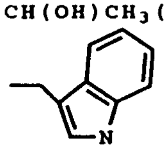 (R) | $SCH_3$ | $CH_3$ | 1 | 1 | - | 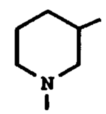 | 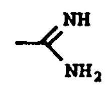 |
| 12 | $CO_2C_6H_5$ | $-CH_2CH_2CO_2H$(R) | H | $CH_3$ | 0 | 0 | CO | 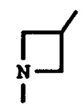 | 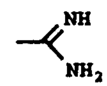 |

| Example No. | $R^3$ | $R^4$ | $R^2$ | $R^1$ | n | p | Q | A | $R^5$ |
|---|---|---|---|---|---|---|---|---|---|
| 13 | $CO_2CH_2C_6H_5$ | $CH_2OCH_2Ph$ (R) | H | H | 1 | 0 | - | 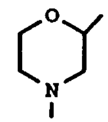 | 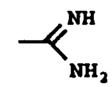 |
| 14 | H | $CH_2CH_2Ph$ (S) | H | H | 1 | 1 | - | 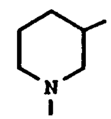 | 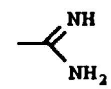 |
| 15 | H | 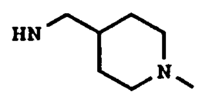 | H | H | 1 | 2 | - | 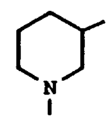 | 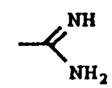 |

## Claims

1. A compound having the structure

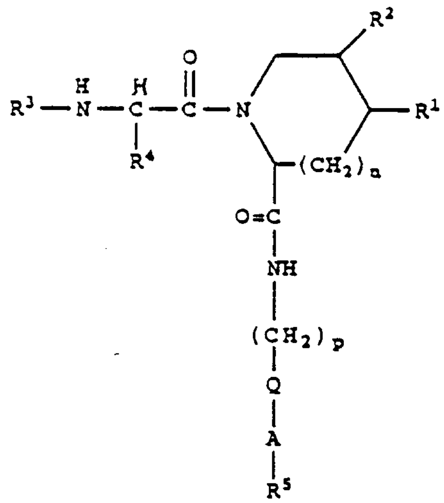

including all stereoisomers, wherein n is 0, I or 2;

p is 0, I or 2;

Q is a single bond or C = O;

A is aryl or cycloalkyl, or an azacycloalkyl ring of 3 to 7 carbons in the ring or an azaheteroalkyl ring of 4 to 6 carbons in the ring as given by the structure

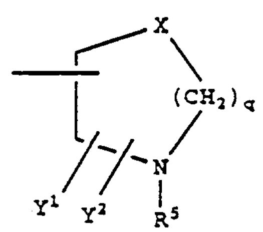

q is 0, I, 2, 3 or 4;

X is $CH_2$, O, S or NH;

q = 0, I, 2, 3 or 4 if X = $CH_2$;

q = 2,3 or 4 if X = O, S, NH; and

$Y^1$ and $Y^2$ are independently H, alkyl, halo or keto; or

$R^5$ is guanidine, amidine or aminomethyl, and

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ together with the carbons to which they are attached form a cycloalkyl, aryl or heteroaryl ring;

$R^4$ is hydrogen, hydroxyalkyl, aminoalkyl, alkyl, cycloalkyl, aryl, arylalkyl, alkenyl, alkynyl, amidoalkyl, arylalkoxyalkyl or an amino acid side chain, either protected or unprotected; and

$R^3$ is hydrogen,

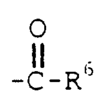

or $-CO_2R^6$, wherein $R^6$ is lower alkyl, aryl, arylalkyl or cycloheteroalkyl; with the provisos that when A is aryl or cycloakyl, $R^5$ is guanidine, amidine or aminomethyl;

where A is azacycloalkyl or azaheteroalkyl, $R^5$ is amidine;

where X is a hetero atom, then there must be at least a 2 carbon chain between X and any N atom in the ring or outside of the ring;

including pharmaceutically acceptable salts thereof.

**2**. The compound as defined in Claim I wherein Q is a single bond.

**3**. The compound as defined in Claim I wherein A is aryl or cycloalkyl.

**4**. The compound as defined in Claim I wherein A is azacycloalkyl or azaheteroalkyl.

**5**. The compound as defined in Claim 4 wherein q is I or 2.

**6**. The compound as defined in Claim I wherein n is 0 or I.

**7**. The compound as defined in Claim I wherein $R^4$ is aralkyl or hydroxyalkyl.

**8**. The compound as defined in Claim I wherein $R^3$ is H.

**9**. The compound as defined in Claim I wherein $R^3$ is H, $R^4$ is aralkyl, $R^1$ and $R^2$ are each H, n is 0 or I.

**10**. The compound as defined in Claim 9 wherein p is I or 2, Q is a single bond, A is azacycloalkyl and $R^5$ is amidine.

**11**. The compound as defined in Claim I wherein $R^3$ is H, $R^4$ is benzyl, $R^1$ and $R^2$ are each H, n is 0, p is I, Q is a single bond, $AR^5$ is

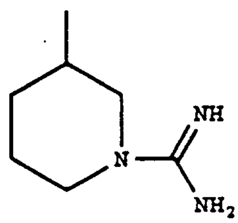

or

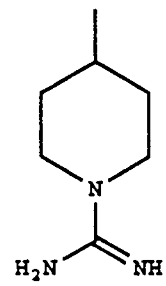

**12**. The compound as defined in Claim I having the structure

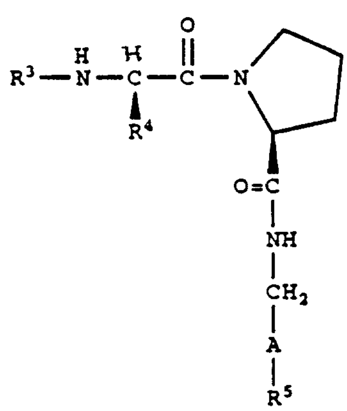

**13**. The compound as defined in Claim I2 wherein $R^4$ is benzyl, and $AR^5$ is

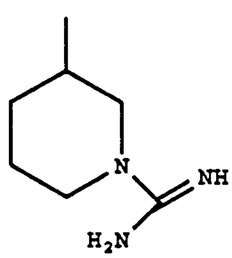

or

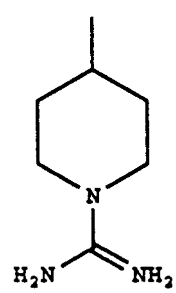

**14**. The compound as defined in Claim I which is N-[[I-(aminoiminomethyl)-4-piperidinyl]methyl]-I-D-phenylalanyl-L-prolinamide.

**15**. The compound as defined in Claim I which is N-[[I-(aminoiminomethyl)-3-piperidinyl]methyl]-I-D-phenylalanyl-L-prolinamide.

**17**. A pharmaceutical composition comprising a compound as defined in Claim I and a pharmaceutically acceptable carrier therefor.

European Patent Office

# EUROPEAN SEARCH REPORT

Application Number
EP 94 11 2916

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,Y | EP-A-0 601 459 (BRISTOL-MYERS SQUIBB CO) 15 June 1994<br>* the whole document *<br>--- | 1-17 | C07K5/06<br>A61K38/55 |
| E | EP-A-0 623 596 (BRISTOL-MYERS SQUIBB CO) 9 November 1994<br>* the whole document *<br>--- | 1-17 | |
| X | WISS. BEITR. MARTIN-LUTHER UNIV. HALLE-WITTENBERG: ENZYMES AND HORMONES IN MEDICAL RESEARCH AND DIAGNOSTICS, vol.52, 1983, HALLE<br>pages 128 - 131<br>U.GRIESSBACH ET AL 'Peptide aldehydes as inhibitors of ptoteolytic enzymes - synthetic aspects'<br>*compound L-Prolinamide, N-[(phenylmethoxy)carbonyl]-D-phenylalanyl-N-[1-(aminoiminomethyl)-2-oxo-3-piperidinyl]-, (S), RN=95925-19-2* | 1,2,4-9 | |
| A | --- | 1-17 | **TECHNICAL FIELDS SEARCHED (Int.Cl.6)** |
| X | SYMP. BIOL. HUNGARY. (PROTEINASE ACTION), vol.25, 1984, BUDAPEST<br>pages 277 - 298<br>S BAJUSZ 'Peptide inhibitors of Trypsin-like enzymes'<br>*see Table 5, page 289, last two entries* | 1,3,6-9, 12 | C07K<br>A61K<br>C07D |
| Y | --- | 1-17 | |
| Y | FOLIA HAEMATOL, vol.109, no.1, 1982, LEIPZIG<br>pages 16 - 21<br>S BAJUSZ 'Design and synthesis of peptide inhibitors of blood coagulation'<br>* the whole document *<br>---<br>-/-- | 1-17 | |

The present search report has been drawn up for all claims

| Place of search | Date of completion of the search | Examiner |
|---|---|---|
| MUNICH | 21 December 1994 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 11 2916

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP-A-0 504 064 (MERRELL DOW PHARMACEUTICALS INC.) 16 September 1992<br>* the whole document *<br>--- | 1-17 | |
| Y | WO-A-93 11152 (AKTIEBOLAGET ASTRA) 10 June 1993<br>* the whole document *<br>----- | 1-17 | |

TECHNICAL FIELDS SEARCHED (Int.Cl.6)

The present search report has been drawn up for all claims

| Place of search | Date of completion of the search | Examiner |
|---|---|---|
| MUNICH | 21 December 1994 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document